# EUROPÄISCHE PATENTSCHRIFT

(11) **EP 3 678 603 B1**
(45) Veröffentlichungstag und Bekanntmachung des Hinweises auf die Patenterteilung: **24.09.2025**
(21) Anmeldenummer: 18733230.9
(22) Anmeldetag: 19.06.2018
(51) Int. Cl.: A61F 2/60, A61F 2/68, A61F 2/64, A61F 2/66, F16D 41/066

(54) **KLEMMROLLENGESPERRE**
FREEWHEEL CLUTCH
EMBRAYAGE UNIDIRECTIONNEL

(30) Priorität: 06.09.2017 DE 102017120466
(43) Veröffentlichungstag der Anmeldung: 15.07.2020
(73) Patentinhaber: Otto Bock Healthcare Products GmbH, 1110 Wien (AT)
(72) Erfinder: SAGMEISTER, Luis, 2823 Pitten (AT); LEDINGER, Christoph, 2514 Möllersdorf (AT)
(74) Vertreter: Gramm, Lins & Partner Patent- und Rechtsanwälte PartGmbB
(86) Internationale Anmeldenummer: PCT/EP2018/066286
(87) Internationale Veröffentlichungsnummer: WO 2019/048098

(56) Entgegenhaltungen:
- DE-A1- 102006 046 495
- GB-A- 495 281
- US-A1- 2002 148 696
- US-A1- 2002 148 696
- US-A1- 2017 165 088
- US-B1- 8 715 367

## Beschreibung

Die Erfindung betrifft ein Klemmrollengesperre mit einem Gehäuse und mindestens zwei, vorzugsweise mindestens drei Klemmscheiben, die in dem Gehäuse drehbar angeordnet sind und jeweils wenigstens eine Ausnehmung, in der sich eine Klemmrolle befindet, wenigstens einen Antriebsvorsprung und wenigstens einen Abtriebsvorsprung aufweisen, wobei die Klemmscheiben derart angeordnet und ausgebildet sind, dass ein Drehen der Klemmscheiben durch Antreiben der Antriebsvorsprung möglich ist und beim Antreiben der Abtriebsvorsprünge ein Drehen der Klemmscheiben verhindert wird.

Ein solches Klemmrollengesperre ist beispielsweise aus der DE 10 2006 046 495 A1 bekannt. Die US 2002/0148696 A1 beschreibt eine besondere Art eines herkömmlichen Freilaufs, bei dem die Rotation in eine Richtung möglich und in die entgegengesetzte Richtung unmöglich ist. Dies ist unabhängig davon, ob Antriebsvorsprünge oder Abtriebsvorsprünge angetrieben werden.

Gesperre und ganz besondere Klemmrollengesperre und andere Art von Freiläufen werden in einer Vielzahl unterschiedlicher Anwendungen, beispielsweise bei orthopädietechnischen Einrichtungen, verwendet, wenn eine Rotation zweier Bauteile relativ zueinander nicht in allen Richtungen oder nicht von allen Seiten angetrieben erlaubt werden soll. So wird beispielsweise bei Prothesen der oberen Extremität das Greifen, Heben oder Drehen von Endeffektoren meist mittels Elektromotoren und entsprechenden Getrieben realisiert. In den meisten dieser Fälle soll die einmal aufgebrachte Kraft, beispielsweise eine Griffkraft, auch nach dem Ausschalten des Stroms des Elektromotors aufrechterhalten bleiben. Dies soll jedoch erreicht werden, ohne dass es notwendig ist, den Strom aufrechtzuerhalten, um Energie zu sparen. Üblicherweise wird diese Aufgabe durch ein Gesperre gelöst, welches den Antriebsstrang gegen Betätigung durch externe Kräfte blockiert. Dabei ist diese Blockierung für manche Anwendungen unabhängig von der externen Kraft, während bei anderen Anwendungen nur in eine Drehrichtung eine Blockade erreicht werden soll. Bei Prothesen der oberen Extremität können diese Kräfte beispielsweise durch das Halten eines Gegenstandes oder dadurch hervorgerufen werden, dass sich der Träger der orthopädietechnischen Einrichtung, vorwiegend also der Prothese, auf dem Greifgerät abstützt. Gleichzeitig muss jedoch erreicht werden, dass der Antriebsstrang motorseitig frei laufen kann. Wird er durch den Motor, beispielsweise den Elektromotor, angetrieben, soll kein zusätzlicher Widerstand einer Bewegung des Endeffektors entgegenstehen. Die Verbindung in einer Arm- oder Handprothese sind jedoch nur zwei Beispiele einer ganzen Reihe von Anwendungsmöglichkeiten, insbesondere im Bereich der orthopädietechnischen Einrichtungen. So können auch Gelenke von Orthesen, andere Bauteile von Orthesen oder andere Prothesen mit entsprechenden Klemmrollengesperren ausgebildet sein.

Aus dem Stand der Technik sind Handprothesen bekannt, bei denen zur Erreichung der gewünschten Blockierung des Antriebsstranges gegen Betätigung durch externe Kräfte ein Schlingfederngesperre verwendet wird, bei welchem es durch Verdrehen zweier Federenden zu einer Durchmesseränderung und somit zu einem Reibschluss eines feststehenden Gehäuses führt. Durch diesen Reibschluss wird die gewünschte Sperrwirkung aufgebaut. Dies ist beidseitig sperrend oder beidseitig freilaufend ausführbar. In anderen Produkten und orthopädietechnischen Einrichtungen kommen oftmals vereinfachte Variationen von Klemmrollengesperren zum Einsatz, welche üblicherweise nur in eine Drehrichtung sperren und in die Gegenrichtung einen Antrieb ermöglichen. Dabei ist es oftmals unerheblich, von welcher Seite das Klemmrollengesperre angetrieben wird, ob also die wirkende Kraft oder das wirkende Drehmoment durch eine Antriebseinrichtung, insbesondere den Elektromotor, oder durch eine externe Kraft oder ein externes Drehmoment aufgebracht wird.

Aus der GB 495 281 A ist ein künstliches Kniegelenk bekannt, bei dem eine Trommelbremse mit einem Freilaufmechanismus kombiniert ist. Dieser Freilaufmechanismus weist eine Klemmrollensperre mit einer Klemmscheibe auf, die in einem Gehäuse drehbar angeordnet ist. Die Rotation ist in einer Richtung möglich, hierbei werden Klemmelemente mit einem inneren Element zusammen angetrieben. In die andere Rotationsrichtung hingegen aktiviert die Bremse.

Die US 2002/148696 A1 betrifft eine Freilaufkupplung mit einem durch schichtweise ineinander gepresste Ringe aufgebauten Klemmengehäuse, in dem eine Achse drehbar gelagert ist.

Der Erfindung liegt daher die Aufgabe zugrunde, ein Klemmrollengesperre vorzuschlagen, das die gewünschten Sperrwirkungen aufweist und gleichzeitig kostengünstig, schnell und einfach herzustellen ist.

Die Erfindung löst die gestellte Aufgabe durch ein Klemmrollengesperre gemäß des Anspruchs 1.

Über die Anzahl der verwendeten Ausnehmungen und Klemmrollen lässt sich die Sperrkraft nahezu beliebig skalieren.

Vorteilhafterweise erstreckt sich wenigstens eine der Klemmrollen, bevorzugt jedoch alle Klemmrollen, durch die Ausnehmung wenigstens einiger, bevorzugt jedoch aller Klemmscheiben.

Erfindungsgemäß sind die Ausnehmungen der Klemmscheiben nach radial außen offen und auf dieser Seite vom Gehäuse begrenzt. Dabei verfügen sie über eine Freilaufseite, die so groß ist, dass eine Klemmrolle nicht mit dem Gehäuse in Kontakt kommt, und eine Sperrseite, die so klein ist, dass eine Klemmrolle mit dem Gehäuse in Kontakt kommt.

Das Wirkprinzip eines Klemmrollengesperres ist aus dem Stand der Technik seit langem bekannt. Die wenigstens eine Klemmrolle befindet sich in der wenigstens einen Ausnehmung, die nach radial außen offen und vom Gehäuse begrenzt ist. Wird nun die Klemmscheibe gedreht, bewegt sich die Klemmrolle relativ zur Klemmscheibe innerhalb der Ausnehmung. Bewegt sie sich dabei auf die Freilaufseite der Ausnehmung, kommt sie in diesem Fall mit dem Gehäuse nicht in Kontakt, sondern kann sich frei auf der Freilaufseite der Ausnehmung bewegen. Eine Drehung der Klemmscheibe relativ zum Gehäuse ist auf diese Weise möglich. Bewegt sich die Klemmrolle jedoch auf die Sperrseite der Ausnehmung, kommt sie aufgrund der geringen Größe der Ausnehmung auf dieser Seite mit dem Gehäuse in Kontakt und wird zwischen dem Grund der Ausnehmung, also der Klemmrolle, und dem Gehäuse eingeklemmt, sodass eine weitere Rotation der Klemmscheibe relativ zum Gehäuse nicht möglich ist.

Erfindungsgemäß liegt für einen Teil der Klemmscheiben die Freilaufseite im Uhrzeigersinn vor der Sperrseite und für einen anderen Teil der Sperrscheiben im Uhrzeigersinn hinter der Sperrseite. Damit unterscheidet sich ein entsprechendes Klemmrollengesperre von Klemmrollengesperren aus dem Stand der Technik. Bei diesen ist in der Regel nur eine Klemmscheibe oder eine Mehrzahl identischer Klemmscheiben vorhanden, sodass die Freilaufseite aller Klemmscheiben beispielsweise im Uhrzeigersinn ausschließlich vor oder ausschließlich hinter der Sperrseite liegt. Dadurch wird erreicht, dass eine Rotation der Klemmscheiben im Gehäuse in eine Richtung möglich ist, wenn durch die Rotation die Klemmrolle in die Freilaufseite der Ausnehmung bewegt wird, und eine Rotation der Klemmscheiben im Gehäuse in die entgegengesetzte Richtung durch die Klemmrolle blockiert wird, die sich durch die Bewegung auf die Sperrseite der Ausnehmung bewegt und so zwischen der Klemmscheibe und dem Gehäuse eingeklemmt wird. Ein gleichzeitiges Antreiben oder Blockieren aller Klemmscheiben ist bei hier beschriebenen Klemmrollengesperren dieser Ausführungsform nicht möglich.

In einer bevorzugten Ausgestaltung werden durch ein Antreiben der Antriebsvorsprünge in einer Drehrichtung nur die Klemmscheiben angetrieben, für die die Freilaufseite in Drehrichtung hinter der Sperrseite liegt. Bei diesen Klemmscheiben bewegt sich durch diesen Antrieb folglich die Klemmrolle in die Freilaufseite der Ausnehmung, sodass durch diese Klemmscheiben eine Rotation nicht beeinträchtigt wird. Da dies jedoch vorzugsweise nur für einen Teil der Klemmscheiben der Fall ist, wird auch nur dieser Teil der Klemmscheiben angetrieben. Der andere Teil der Klemmscheiben, bei dem sich für die gegebene Drehrichtung die Freilaufseite in Drehrichtung vor der Sperrseite befindet, wird nicht über die Antriebsvorsprünge oder die Abtriebsvorsprünge angetrieben, sondern wird über die Klemmrolle bewegt, die in diesem Fall als Mitnehmer wirkt. Dabei befindet sich die Klemmrolle für diesen Teil der Sperrscheiben im in Drehrichtung vorderen Teil der Ausnehmung, der für diesen Teil der Klemmscheiben die Freilaufseite ist. Auf diese Weise ist gewährleistet, dass unabhängig von der Drehrichtung eine Rotation immer möglich ist, solange der Antrieb über die Antriebsvorsprünge erfolgt. Die Antriebsvorsprünge des ersten Teils der Klemmscheiben, sind dabei vorzugsweise versetzt zu den Antriebsvorsprüngen des anderen Teils der Sperrscheiben angeordnet, sodass ein entsprechender antriebsseitiger Mitnehmer, der beispielsweise mit einem Elektromotor oder einer anderen Antriebseinrichtung gekoppelt ist, nur die jeweils gewünschten Antriebsvorsprünge erreicht und die entsprechenden Klemmscheiben antreiben kann.

Vorteilhafterweise werden durch das Antreiben der Abtriebsvorsprünge in einer Drehrichtung nur die Klemmscheiben angetrieben, für die die Sperrseite in Drehrichtung hinter der Freilaufrichtung liegt. Durch diesen Antrieb und die entsprechende Bewegung der Sperrscheiben bewegt sich die Klemmrolle in die Sperrseite der Ausnehmung, sodass eine weitere Rotation verhindert wird, da die Klemmrolle zwischen dem Grund der Ausnehmung und dem Gehäuse eingeklemmt ist. Auch dies geschieht unabhängig von der Drehrichtung, da je nach Drehrichtung der eine Teil oder der andere Teil der Sperrscheiben über die Abtriebsvorsprünge angetrieben wird. Auch die Abtriebsvorsprünge der beiden Teile der Klemmscheiben sind folglich vorzugsweise versetzt zueinander angeordnet.

Es hat sich als vorteilhaft herausgestellt, wenn das Klemmrollengesperre eine ungerade Anzahl von Klemmscheiben und/oder wenigstens drei Ausnehmungen pro Klemmscheibe aufweist. In diesen wenigstens drei Ausnehmungen befindet sich vorteilhafterweise jeweils wenigstens eine Klemmrolle. Je mehr Ausnehmungen und Klemmrollen vorhanden sind, desto größer ist die Sperrkraft, die erreicht werden kann. In einer konstruktiv besonders einfachen Ausgestaltung des Klemmrollengesperres sind die einzelnen Klemmscheiben identisch ausgebildet und werden lediglich versetzt und gegebenenfalls gedreht zueinander angeordnet. Auf diese Weise wird die Anzahl der unterschiedlichsten Bauteile, die zur Konstruktion des Klemmrollengesperres nötig sind, reduziert.

In einer bevorzugten Ausgestaltung bestehen die Klemmscheiben aus einem metallischen Werkstoff. Alternativ oder zusätzlich können auch Kunststoffe, Holz oder Holzwerkstoffe oder andere Materialien verwendet werden. Sie können beispielsweise extrudiert, gestanzt, lasergeschnitten, wasserstrahlgeschnitten, gesägt oder spritzgussgefertigt sein.

Die Erfindung löst die gestellte Aufgabe zudem durch eine orthopädietechnische Einrichtung mit einer Antriebseinrichtung, die mit wenigstens einem antriebsseitigen Mitnehmer gekoppelt ist, und einem Endeffektor, der mit einem abtriebsseitigen Mitnehmer gekoppelt ist, und mit einem Klemmrollengesperre der hier beschriebenen Art, wobei der antriebsseitige Mitnehmer mit den Antriebsvorsprüngen und der abtriebsseitige Mitnehmer mit den Abtriebsvorsprüngen in Eingriff steht oder bringbar ist.

Soll der Endeffektor über die Antriebseinrichtung angetrieben werden, wird zunächst über die Antriebseinrichtung der antriebsseitige Mitnehmer in Rotation versetzt. Dieser ist mit den Antriebsvorsprüngen gekoppelt, sodass bevorzugt nur die Klemmscheiben angetrieben werden, für die die Freilaufseite in Drehrichtung hinter der Sperrseite liegt. Dies führt dazu, dass eine Rotation der Klemmscheiben im Gehäuse ohne zusätzlichen Widerstand möglich ist. Dadurch wird auch der abtriebsseitige Mitnehmer angetrieben und das von der Antriebseinrichtung aufgebrachte Drehmoment auf den Endeffektor übertragen.

Wird hingegen von außen ein Drehmoment oder eine Kraft auf den Endeffektor aufgebracht, wird dieses Drehmoment oder diese Kraft auf den abtriebsseitigen Mitnehmer übertragen, der mit den Abtriebsvorsprüngen in Eingriff steht oder mit diesen in Eingriff bringbar ist. Beim Antreiben dieser Abtriebsvorsprünge wird jedoch vorteilhafterweise nur der Teil der Klemmscheiben angetrieben, für die die Sperrseite in Drehrichtung hinter der Freilaufseite liegt. Dies führt, wie bereits dargelegt, zu einer Sperrung des Klemmrollengesperres.

Mithilfe der beiliegenden Zeichnungen wird nachfolgend ein Ausführungsbeispiel der vorliegenden Erfindung näher erläutert. Es zeigt
- Figur 1 -: eine Klemmscheibe für ein Klemmrollengesperre gemäß einem ersten Ausführungsbeispiel der vorliegenden Erfindung,
- Figur 2 -: einen Teil einer orthopädietechnischen Einrichtung in einer Explosionsdarstellung,
- Figur 3 -: die Draufsicht auf eine Abtriebsseite der Vorrichtung aus Figur 2,
- Figur 4 -: die Draufsicht auf eine Antriebsseite der Vorrichtung aus Figur 2,
- Figur 5 -: zwei Detailansichten eines Klemmrollengesperres und
- Figur 6 -: eine orthopädietechnische Einrichtung gemäß einem weiteren Ausführungsbeispiel der vorliegenden Erfindung in einer Explosionsdarstellung.

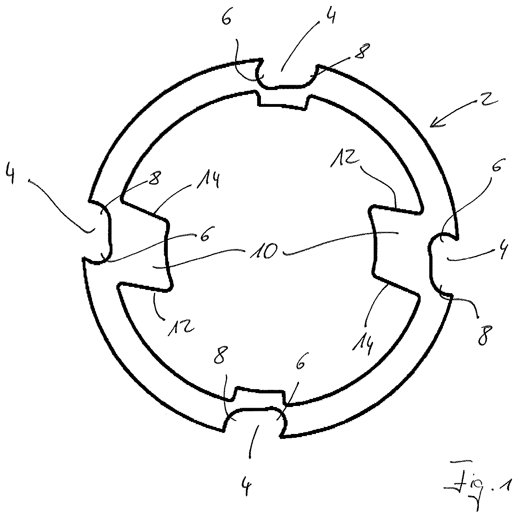

Figur 1 zeigt eine Klemmscheibe 2 für ein Klemmrollengesperre gemäß einem ersten Ausführungsbeispiel der vorliegenden Erfindung. Die Klemmscheibe 2 verfügt über vier Ausnehmungen 4, die im gezeigten Ausführungsbeispiel äquidistant über den Umfang verteilt sind. Jede der Ausnehmungen verfügt über eine Freilaufseite 6 und eine Sperrseite 8. Die Ausnehmungen 4 sind nach radial außen geöffnet und werden im montierten Zustand eines Klemmrollengesperres durch ein Gehäuse begrenzt.

Nach radial innen springen zwei Vorsprünge 10 hervor, von denen jeweils eine Seite einen Antriebsvorsprung 12 und die gegenüberliegende Seite einen Abtriebsvorsprung 14 bildet. Selbstverständlich können Antriebsvorsprung 12 und Abtriebsvorsprung 14 auch getrennte Vorsprünge sein.

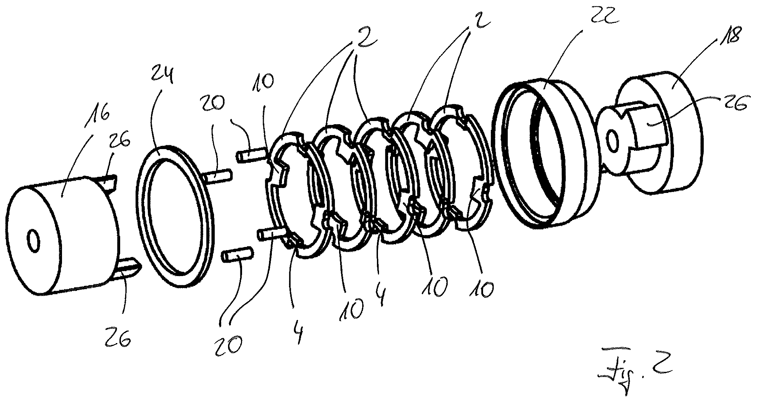

Figur 2 zeigt eine Explosionsdarstellung eines Klemmrollengesperres gemäß einem Ausführungsbeispiel der vorliegenden Erfindung mit einem antriebsseitigen Mitnehmer 16 und einem abtriebsseitigen Mitnehmer 18. Das Klemmrollengesperre verfügt über fünf Klemmscheiben 2, die jeweils vier Ausnehmungen 4 aufweisen, wie sie in Figur 1 dargestellt sind. Darin befinden sich vier Klemmrollen 20. Die Klemmscheiben 2 mit den Klemmrollen 20 befinden sich in einem Gehäuse 22, das die Ausnehmungen 4 nach radial außen begrenzt. Eine Deckscheibe 24 verhindert im gezeigten Ausführungsbeispiel, dass die Klemmrollen 20 in axialer Richtung aus den Ausnehmungen 4 herauswandern können.

Sowohl der antriebsseitige Mitnehmer 16 als auch der abtriebsseitige Mitnehmer 18 verfügen über Mitnehmerklauen 26, mit denen sie mit den Antriebsvorsprüngen 12 oder den Abtriebsvorsprüngen 14 in Eingriff stehen oder in Eingriff gebracht werden können.

Man erkennt bereits in Figur 2, dass die Klemmscheiben 2 versetzt zueinander angeordnet sind, sodass ein Winkel zwischen den Vorsprüngen 10 zweier benachbarter Klemmscheiben 2 im gezeigten Ausführungsbeispiel 90° beträgt.

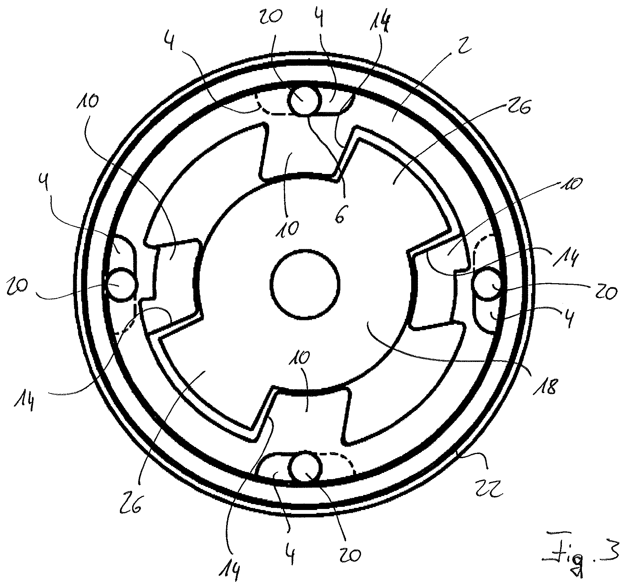

Figur 3 zeigt eine Draufsicht auf eine Abtriebsseite einer derartigen Vorrichtung. Man erkennt den abtriebsseitigen Mitnehmer 18 mit den beiden Mitnehmerklauen 26 und eine Klemmscheibe 2 mit zwei Vorsprüngen 10. Die beiden Mitnehmerklauen 26 des abtriebsseitigen Mitnehmers 18 können mit dem jeweiligen Abtriebsvorsprung 14 der Vorsprünge 10 in Eingriff gebracht werden, indem der abtriebsseitige Mitnehmer 18 in Rotation versetzt wird.

In Figur 3 sind zudem zwei weitere Vorsprünge 10 dargestellt, die sich in Figur 3 rechts und links befinden. Diese gehören zu einer Klemmscheibe 2, die hinter der dargestellten Klemmscheibe 2 angeordnet ist. Auch diese Vorsprünge 10 verfügen über Abtriebsvorsprünge 14, mit denen der abtriebsseitige Mitnehmer 18 über die Mitnehmerklauen 26 in Eingriff gebracht werden kann.

Die vordere gezeigte Klemmscheibe 2 verfügt über die vier Ausnehmungen 4, in denen jeweils eine Klemmrolle 20 angeordnet ist, die sich im gezeigten Ausführungsbeispiel jeweils in der Freilaufseite 6 befindet. Mit einer gestrichelten Linie sind die Ausnehmungen 4 der hinter der Sperrscheibe 2 liegenden weiteren Sperrscheibe 2 dargestellt. Auch für diese Ausnehmungen 4 befindet sich die jeweilige Klemmrolle 20 an der Freilaufseite 6.

Wird nun im gezeigten Ausführungsbeispiel der abtriebsseitige Mitnehmer 18 in Rotation versetzt, kommen die Mitnehmerklauen 26 entweder mit den Abtriebsvorsprüngen 14 der vorderen Klemmscheibe 2 oder mit den Abtriebsvorsprüngen 14 der dahinterliegenden Klemmscheibe 2 in Eingriff. Wird der abtriebsseitige Mitnehmer 18 beispielsweise gegen den Uhrzeigersinn gedreht, kommen seine Mitnehmerklauen 26 mit dem Abtriebsvorsprüngen 14 der vordersten Klemmscheibe 2 in Eingriff. Diese wird folglich ebenfalls gegen den Uhrzeigersinn gedreht, wodurch die Klemmrollen 20 in den jeweiligen Ausnehmungen 4 auf die jeweilige Sperrseite 8 bewegt werden. Dadurch kommt es zu einer Sperrung des Klemmrollengesperres, da die Klemmrollen 20 zwischen dem Boden oder dem Grund der Ausnehmung 4 und dem Gehäuse 22 eingeklemmt werden.

Wird hingegen der abtriebsseitige Mitnehmer 18 im Uhrzeigersinn gedreht, kommen seine Mitnehmerklauen 26 mit den Abtriebsvorsprüngen 14 der dahinterliegenden Klemmscheibe in Eingriff. Diese wird dadurch ebenfalls im Uhrzeigersinn bewegt, wodurch wieder die Klemmrollen 20 in den Ausnehmungen 4 dieser Klemmscheibe 2 von der Freilaufseite 6 auf die Sperrseite 8 bewegt werden, sodass es auch hier zu einer Sperrung kommt. Unabhängig davon, in welche Richtung der abtriebsseitige Mitnehmer 18 folglich bewegt wird, kommt es immer zu einer Sperrung des Klemmrollengesperres.

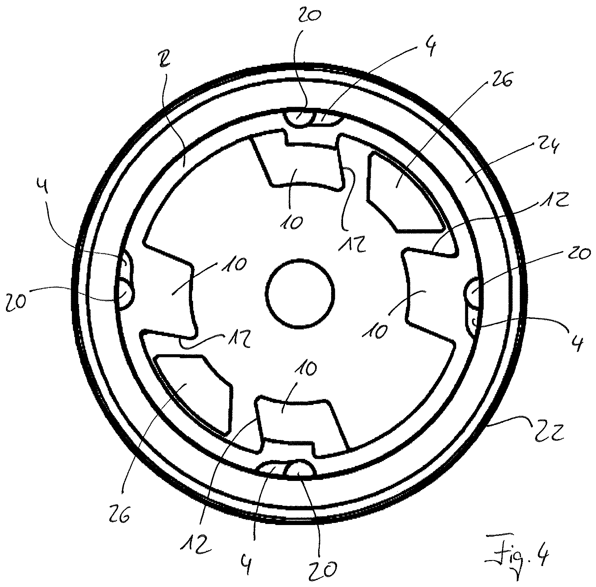

Figur 4 zeigt eine Draufsicht auf die Antriebsseite eines derartigen Klemmrollengesperres. Man erkennt die Ausnehmungen 4, in denen sich jeweils eine Klemmrolle 20 befindet, die im gezeigten Ausführungsbeispiel jedoch teilweise von der Deckscheibe 24 abgedeckt sind. Die Klemmrollen 20 befinden sich jeweils auf der Freilaufseite 6 der Ausnehmung 4. Die beiden Mitnehmerklauen 26 des antriebsseitigen Mitnehmers 16 sind ebenfalls dargestellt. Die beiden rechts und links dargestellten Vorsprünge 10 gehören zu der in dieser Richtung vordersten Klemmscheibe 2, währende die oben und unten dargestellten Vorsprünge 10 zur dahinterliegenden Klemmscheibe gehören. Die Vorsprünge 10 verfügen jeweils über einen Antriebsvorsprung 12, der mit der jeweiligen Mitnehmerklaue 26 in Eingriff bringbar ist.

Wird der antriebsseitige Mitnehmer 16 mit seinen Mitnehmerklauen 26 im Uhrzeigersinn bewegt, kommen die Mitnehmerklauen 26 mit den Antriebsvorsprüngen 12 der in Figur 4 rechts und links dargestellten Vorsprünge 10 in Eingriff. Auch diese vorderste Klemmscheibe 2, zu der diese Vorsprünge 10 gehören, wird folglich im Uhrzeigersinn bewegt, sodass die Klemmrollen 20 auf den Freilaufseiten 6 der Ausnehmungen 4 verbleiben. Eine Rotation ist daher möglich.

Wird der antriebsseitige Mitnehmer 16 mit seinem Mitnehmerklauen 26 jedoch gegen den Uhrzeigersinn bewegt, kommen die Mitnehmerklauen 26 mit den Antriebsvorsprüngen 12 der in Figur 4 oben und unten dargestellten Vorsprünge in Eingriff, die zu der hinter der gezeigten Klemmscheibe 2 liegenden Klemmscheibe gehören. Wie in Figur 4 gezeigt, befindet sich für diese Klemmscheibe 2 die Sperrseite 8 der Ausnehmung 4 für diese Drehrichtung gegen den Uhrzeigersinn vor der entsprechenden Freilaufseite 6, sodass auch in dieser Drehrichtung eine Rotation der Klemmscheiben 2 relativ zum Gehäuse 22 möglich ist.

Unabhängig von der Richtung, in der der antriebsseitige Mitnehmer 16 gedreht wird, ist eine Rotation folglich immer möglich.

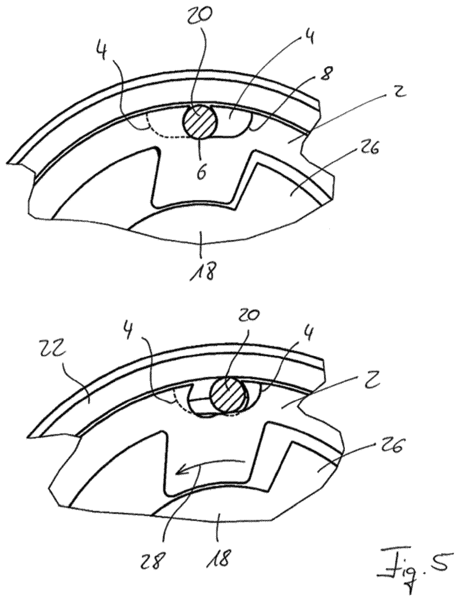

Dies wird in Figur 5 deutlich. Im oberen Bereich der Figur 5 ist eine Klemmrolle 20 dargestellt, die sich in einer Ausnehmung 4 der vorderen Klemmscheibe 2 befindet, wobei die Ausnehmung 4 mit einer durchgezogenen Linie dargestellt ist. Für diese Ausnehmung 4 befindet sich auf der rechten Seite der Ausnehmung 4 die Sperrseite 8 und auf der linken Seite die Freilaufseite 6, in der sich die Klemmrolle 20 befindet. Für die dahinterliegende Klemmscheibe 2 ist mit einer gestrichelten Linie ebenfalls die Ausnehmung 4 dargestellt, die nun jedoch andersherum orientiert ist. Die Freilaufseite 6 befindet sich rechts, während sich die Sperrseite 8 links befindet. Man erkennt zudem eine Mitnehmerklaue 26 des abtriebsseitigen Mitnehmers 18.

Im unteren Bereich der Figur 5 ist dargestellt, was passiert, wenn die vordere Klemmscheibe 2 durch den abtriebsseitigen Mitnehmer 18 entlang des Pfeils 28, im gezeigten Ausführungsbeispiel also gegen den Uhrzeigersinn, in Rotation versetzt wird. Die beiden Ausnehmungen 4, die wieder durch die durchgezogene Linie für die vorderste Klemmscheibe 2 und durch die gestrichelte Linie für die dahinterliegende Klemmscheibe dargestellt sind, verschieben sich relativ zueinander, und die Klemmrolle 20 wird in der Ausnehmung 4 der vordersten Klemmscheibe 2 nach rechts, also in Richtung auf die Sperrseite 8 bewegt. Dadurch wird sie zwischen der Klemmscheibe 2 und dem Gehäuse 22 eingeklemmt und eine weitere Rotation der Klemmscheibe 2 relativ zum Gehäuse 22 wird unterbunden.

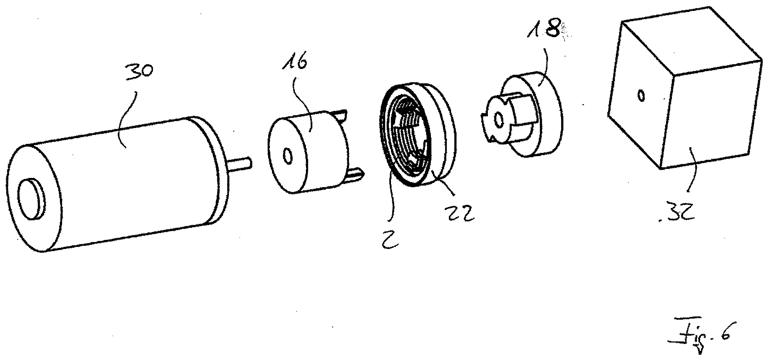

Figur 6 zeigt eine schematische Explosionsdarstellung eines Teils einer orthopädietechnischen Einrichtung mit einer Antriebseinrichtung 30, einem antriebsseitigen Mitnehmer 16, dem Klemmrollengesperre mit dem Gehäuse 22 und den darin angeordneten Klemmscheiben 2 sowie einem abtriebsseitigen Mitnehmer 18, der an einem nur schematisch dargestellten Endeffektor 32 angekoppelt ist.

### Bezugszeichenliste

- 2: Klemmscheibe
- 4: Ausnehmung
- 6: Freilaufseite
- 8: Sperrseite
- 10: Vorsprung
- 12: Antriebsvorsprung
- 14: Abtriebsvorsprung
- 16: antriebsseitiger Mitnehmer
- 18: abtriebsseitiger Mitnehmer
- 20: Klemmrolle
- 22: Gehäuse
- 24: Deckscheibe
- 26: Mitnehmerklaue
- 28: Pfeil
- 30: Antriebseinrichtung
- 32: Endeffektor

## Patentansprüche

1. Klemmrollengesperre mit
einem Gehäuse (22) und
mindestens zwei, vorzugsweise mindestens drei Klemmscheiben (2), die
• in dem Gehäuse (22) drehbar angeordnet sind und
• jeweils wenigstens eine Ausnehmung (4), in der sich eine Klemmrolle (20) befindet, **dadurch gekennzeichnet, dass** die Klemmscheiben
• wenigstens einen Antriebsvorsprung (12) und
• wenigstens einen Abtriebsvorsprung (14) aufweisen,
wobei die Klemmscheiben (2) derart angeordnet und ausgebildet sind, dass ein Drehen der Klemmscheiben (2) durch Antreiben der Antriebsvorsprünge (12) möglich ist und beim Antreiben der Abtriebsvorsprünge (14) ein Drehen der Klemmscheiben (2) verhindert wird, wobei
die Ausnehmungen (4) der Klemmscheiben (2) nach radial außen offen sind und auf dieser Seite vom Gehäuse (22) begrenzt werden, wobei sie eine Freilaufseite (6), die so groß ist, dass eine Klemmrolle (20) nicht mit dem Gehäuse (22) in Kontakt kommt, und eine Sperrseite (8) aufweisen, die so klein ist, dass eine Klemmrolle (20) mit dem Gehäuse (22) in Kontakt kommt, wobei für einen Teil der Klemmscheiben (2) die Freilaufseite (6) im Uhrzeigersinn vor der Sperrseite (8) und für einen anderen Teil der Sperrscheiben (2) im Uhrzeigersinn hinter der Sperrseite (8) liegt .

2. Klemmrollengesperre nach Anspruch 1, **dadurch gekennzeichnet, dass** sich wenigstens eine, bevorzugt alle Klemmrollen (20) durch Ausnehmungen (4) wenigstens einer, vorzugsweise aller Klemmscheiben (2) erstrecken.

3. Klemmrollengesperre nach Anspruch 1 oder 2, **dadurch gekennzeichnet, dass** durch Antreiben der Antriebsvorsprünge (12) in einer Drehrichtung nur die Klemmscheiben (2) angetrieben werden, für die die Freilaufseite (6) in Drehrichtung hinter der Sperrseite (8) liegt.

4. Klemmrollengesperre nach Anspruch 1, 2 oder 3, **dadurch gekennzeichnet, dass** durch Antreiben der Abtriebsvorsprünge (14) in einer Drehrichtung nur die Klemmscheiben (2) angetrieben werden, für die die Sperrseite (8) in Drehrichtung hinter der Freilaufseite (6) liegt.

5. Klemmrollengesperre nach einem der vorstehenden Ansprüche, **dadurch gekennzeichnet, dass** das Klemmrollengesperre eine ungerade Anzahl Klemmscheiben (2) und/oder wenigstens drei Ausnehmungen (4) pro Klemmscheibe (2) aufweist.

6. Klemmrollengesperre nach einem der vorstehenden Ansprüche, **dadurch gekennzeichnet, dass** die Klemmscheiben (2) die gleiche Kontur aufweisen.

7. Klemmrollengesperre nach einem der vorstehenden Ansprüche, **dadurch gekennzeichnet, dass** die Klemmscheiben (2) aus einem metallischen und/oder einem keramischen Werkstoff bestehen.

8. Orthopädietechnische Einrichtung mit einer Antriebseinrichtung (30), die mit wenigstens einem antriebsseitigen Mitnehmer (16) gekoppelt ist, und einem Endeffektor (21), der mit einem abtriebsseitigen Mitnehmer (18) gekoppelt ist, und mit einem Klemmrollengesperre nach einem der vorstehenden Ansprüche, wobei der antriebsseitige Mitnehmer (16) mit den Antriebsvorsprüngen (12) und der abtriebsseitige Mitnehmer (18) mit den Abtriebsvorsprüngen (14) in Eingriff steht oder bringbar ist.

## Claims

1. A jamming roller lock with
a housing (22) and
at least two, preferably at least three, clamping discs (2) which
• are arranged in the housing (22) such that they can be rotated and
• comprise at least one recess (4), in which a jamming roller (20) is situated,
**characterized in that** the clamping discs comprise
• at least one driving projection (12) and
• at least one driven projection (14),
wherein the clamping discs (2) are arranged and designed in such a way that a rotation of the clamping discs (2) is possible by driving the driving projections (12) and a rotation of the clamping discs (2) by driving the driven projections (14) is prevented
wherein the recesses (4) of the clamping discs (2) are open radially outwards and are restricted by the housing (22) on this side, wherein they comprise a freewheel side (6), which is large enough to ensure that a jamming roller (20) does not come into contact with the housing (22), and a locking side (8), which is small enough to ensure that a jamming roller (20) comes into contact with the housing (22), wherein for one part of the clamping discs (2), the freewheel side (6) lies in front of the locking side (8) in the clockwise direction, and for the other part of the locking discs (2) it lies behind the locking side (8) in the clockwise direction.

2. The jamming roller lock according to claim 1, **characterized by the fact that** at least one, preferably all, jamming rollers (20) extend through recesses (4) of at least one, preferably all, clamping discs (2).

3. The jamming roller lock according to claim 1 or 2, **characterized by the fact that,** by driving the driving projections (12) in one direction of rotation, only those clamping discs (2) are driven for which the freewheel side (6) lies behind the locking side (8) in the direction of rotation.

4. The jamming roller lock according to claim 1, 2 or 3, **characterized by the fact that,** by driving the driven projections (14) in one direction of rotation, only those clamping discs (2) are driven for which the locking side (8) lies behind the freewheel side (6) in the direction of rotation.

5. The jamming roller lock according to one of the above claims, **characterized by the fact that** the jamming roller lock comprises an uneven number of clamping discs (2) and/or at least three recesses (4) per clamping disc (2).

6. The jamming roller lock according to one of the above claims, **characterized by the fact that** the clamping discs (2) have the same contour.

7. The jamming roller lock according to one of the above claims, **characterized by the fact that** the clamping discs (2) are made of a metallic material and/or a ceramic material.

8. An orthopedic device with a driving device (30), which is coupled with at least one driver on the driving side (16), and an end effector (21), which is coupled with a driver on the driven side (18), and a jamming roller lock according to one of the preceding claims, wherein the driver on the driving side (16) is or can be engaged with the driving projections (12) and the driver on the driven side (18) is or can be engaged with the driven projections (14).

## Revendications

1. Mécanisme de blocage à galets de serrage, comprenant
un boîtier (22) et
au moins deux, de préférence au moins trois, rondelles de serrage (2)
• qui sont disposées de manière rotative dans le boîtier (22) et
• qui présentent chacune au moins un évidement (4) dans lequel se trouve un galet de serrage (20),
**caractérisé en ce que**
les rondelles de serrage comprennent
• au moins une saillie menante (12) et
• au moins une saillie menée (14),
sachant que
les rondelles de serrage (2) sont agencées et conçues de telle sorte qu'une rotation des rondelles de serrage (2) est possible par l'entraînement des saillies menantes (12) et que, lors de l'entraînement des saillies menées (14), une rotation des rondelles de serrage (2) est empêchée,
les évidements (4) des rondelles de serrage (2) sont ouverts radialement vers l'extérieur et sont délimités de ce côté par le boîtier (22), en présentant un côté de roue libre (6) qui est suffisamment grand pour qu'un galet de serrage (20) ne vienne pas en contact avec le boîtier (22), et un côté de blocage (8) qui est suffisamment petit pour qu'un galet de serrage (20) vienne en contact avec le boîtier (22),
pour une partie des rondelles de serrage (2), le côté de roue libre (6) est situé devant le côté de blocage (8) dans le sens des aiguilles d'une montre, et pour une autre partie des rondelles de blocage (2), il est situé derrière le côté de blocage (8) dans le sens des aiguilles d'une montre.

2. Mécanisme de blocage à galets de serrage selon la revendication 1, **caractérisé en ce qu'**au moins un, de préférence tous les galets de serrage (20) traverse(nt) les évidements (4) d'au moins une, de préférence de toutes les rondelles de serrage (2).

3. Mécanisme de blocage à galets de serrage selon la revendication 1 ou 2, **caractérisé en ce que** par entraînement des saillies menantes (14) dans un sens de rotation, seules les rondelles de serrage (2) sont entraînées pour lesquelles le côté de roue libre (6) est situé derrière le côté de blocage (8) dans le sens de rotation.

4. Mécanisme de blocage à galets de serrage selon la revendication 1, 2 ou 3,
**caractérisé en ce que** par entraînement des saillies menées (14) dans un sens de rotation, seules les rondelles de serrage (2) sont entraînées pour lesquelles le côté de blocage (8) est situé derrière le côté de roue libre (6) dans le sens de rotation.

5. Mécanisme de blocage à galets de serrage selon l'une des revendications précédentes,
**caractérisé en ce que** le mécanisme de blocage à galets de serrage comprend un nombre impair de rondelles de serrage (2) et/ou au moins trois évidements (4) par rondelle de serrage (2).

6. Mécanisme de blocage à galets de serrage selon l'une des revendications précédentes,
**caractérisé en ce que** les rondelles de serrage (2) ont le même contour.

7. Mécanisme de blocage à galets de serrage selon l'une des revendications précédentes,
**caractérisé en ce que** les rondelles de serrage (2) sont constituées d'un matériau métallique et/ou céramique.

8. Dispositif orthopédique comprenant un dispositif d'entraînement (30) qui est couplé à au moins un entraîneur côté menant (16) et un effecteur terminal (21) qui est couplé à un entraîneur côté mené (18), et comprenant un mécanisme de blocage à galets de serrage selon l'une des revendications précédentes,
dans lequel l'entraîneur côté menant (16) est en prise ou peut être amené en prise avec les saillies menantes (12), et l'entraîneur côté mené (18) est en prise ou peut être amené en prise avec les saillies menées (14).
